(19) **Europäisches Patentamt / European Patent Office / Office européen des brevets**

(11) **EP 4 680 594 B1**

(12) ## EUROPEAN PATENT SPECIFICATION

(45) Date of publication and mention
of the grant of the patent:
**11.03.2026 Bulletin 2026/11**

(21) Application number: **25728808.4**

(22) Date of filing: **30.05.2025**

(51) International Patent Classification (IPC):
**C07C 231/02** *(2006.01)*

(52) Cooperative Patent Classification (CPC):
(C-Sets available)
**C07C 231/02** (Cont.)

(86) International application number:
**PCT/EP2025/064992**

(87) International publication number:
**WO 2025/248088 (04.12.2025 Gazette 2025/49)**

(54) **AMINO ALKYL (METH)ACRYLATES BATCH SYNTHESIS PROCESS**

BATCH-SYNTHESEVERFAHREN FÜR AMINOALKYL(METH)ACRYLATE

PROCÉDÉ DE SYNTHÈSE PAR LOTS D'AMINO ALKYL (MÉTH)ACRYLATES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.05.2024 EP 24179407**

(43) Date of publication of application:
**21.01.2026 Bulletin 2026/04**

(73) Proprietor: **SNF Group**
**42160 Andrézieux-Bouthéon (FR)**

(72) Inventors:
- **LEGRAS, Benoît**
  **42160 ANDREZIEUX-BOUTHEON (FR)**
- **KIEFFER, Johann**
  **42160 ANDREZIEUX-BOUTHEON (FR)**
- **FAVERO, Cédrick**
  **42160 ANDREZIEUX-BOUTHEON (FR)**

(74) Representative: **Ipsilon**
**Le Contemporain**
**50 Chemin de la Bruyère**
**69574 Dardilly Cedex (FR)**

(56) References cited:
**WO-A1-2019/196048     US-A- 4 059 617**

(52) Cooperative Patent Classification (CPC): (Cont.)

C-Sets
**C07C 231/02, C07C 233/20**

## Description

### TECHNICAL FIELD OF THE INVENTION

The present invention relates to a new amino alkyl (meth)acrylates batch synthesis process.

### TECHNICAL BACKGROUND

Amino alkyl (meth)acrylates, such as 2-dimethylaminoethyl (meth)acrylate and its quaternized derivatives, are well-known monomers used in the preparation of polymers used in many industries, such as water treatment, papermaking, home and personal care, or oil & gas industry such as enhanced oil recovery, hydraulic fracturing, water shut-off and others.

These monomers are obtained by transesterification of alkyl(meth)acrylate with an alkanolamine, catalyzed by organometallic derivatives. Choosing the catalyst depends on various criteria, like the nature of the amino alkyl (meth) acrylate or of the alcohol used, or the nature of the synthesis process. The catalyst's role is to displace the equilibrium to produce more amino alkyl (meth)acrylate and reduce the impurities formation.

After the reaction, the catalyst can be recycled in a new synthesis process, but during these recycling cycles, the catalyst tends to be deactivated leading to the formation of more impurities. These impurities, called heavies, are Michael adducts of alcohols and/or amine, such as methanol or dimethylaminoethanol which react on the double bond of the methyl(meth)acrylate, dimethylaminoethyl(meth)acrylate, or (meth)acrylic acid. The formation of these impurities, reduces the reaction yield, increases the wastes generated and decreases the quality of the amino alkyl (meth)acrylate produced which impacts the performances of polymers prepared from such amino alkyl (meth)acrylate.

Many attempts have been made to reduce the formation of heavies. In the document WO2019196048, the applicant proposed to replace part of the original catalyst with a fresh one in the successive synthesis batch, leading to a better lifetime of the recycled catalyst and better yield and purity of the amino alkyl (meth)acrylate produced. However, this solution requires an increase in catalyst consumption.

The document US4059617 describes the synthesis of dimethylaminoethyl methacrylate in batch process by continuous addition of a mixture of dimethylaminoethanol and methylmethacrylate, during the reaction at a rate of about the same as the rate at which the aminoalcohol reactant is consumed. By doing so, the yield is improved and less heavies are formed.

Alternative solutions are disclosed in WO2013045786 and WO2016124837. In these documents heavies undergo thermal cracking in order to reverse their formation. However, such a process requires a lot of energy.

The Applicant has discovered that it is possible to improve the reaction yield and purity of amino alkyl (meth) acrylate obtained during a batch process, to reduce the waste generated, while also having a best recyclability of the catalyst, by starting the synthesis from a specific mixture composition and continuously adding at least one alkanolamine throughout the batch synthesis process. The process according to the invention, also improves the kinetic of the amino alkyl (meth)acrylates synthesis, increases the yield even more while reducing the overall amount of energy required for the synthesis compared to a classical amino alkyl (meth)acrylates synthesis process.

The new amino alkyl (meth)acrylates batch synthesis process of the invention is in line with the principle of environmental consciousness and the impact of industry and man on the planet. The new batch synthesis process improves the yield and purity of the synthetized amino alkyl (meth)acrylates, reduce the wastes generated and allow a best recyclability of the catalyst, leading to an overall reduction of the emission of greenhouse gases, such as $CO_2$, and wastes.

### SUMMARY OF THE INVENTION

The present invention relates to a new amino alkyl (meth)acrylates batch synthesis process.

More specifically, the batch synthesis process of amino alkyl (meth)acrylates comprises the following successive steps:

1) Preparation of a mixture M1 comprising:

(i) from 50 to 97.899% by weight of at least one alkyl$_1$ (meth)acrylate ester;
(ii) from 0.1 to 40% by weight of at least one alkanolamine;
(iii) from 0.001 to 10 % by weight of at least one organometallic catalyst;
(iv) from 2 to 30 % by weight of at least one solvent;

based on the total weight of the mixture M1;
2) preparation of a mixture M2 by:

(i) Heating the mixture M1 to a temperature T1 comprised between 40°C and 120°C and removing the $\text{alkyl}_1$ alcohol that forms during the reaction by azeotropic distillation; the azeotropic distillation is carried out between 30 to 720 min at T1,

(ii) further addition in continuous, during the azeotropic distillation, of a quantity of the at least one alkanolamine comprised between 15 and 85% by weight based on the total amount of alkanolamine used in steps 1) and 2);

3) recovering the amino alkyl (meth)acrylate by distillation of the mixture M2.

the amine of the alkanolamine is a tertiary amine $-NR_1R_2$ wherein $R_1$ and $R_2$, identical or different, are alkyl groups comprising between 1 and 8 carbon atoms and

the $\text{alkyl}_1$ (meth)acrylate ester is linear and the $\text{alkyl}_1$ chain comprises between 1 to 10 carbons.

Another object of the invention relates to the amino alkyl (meth)acrylate obtained from the batch synthesis process according to the invention.

Another object of the present disclosure concerns a polymer obtained from the polymerization of the amino alkyl (meth)acrylate obtained from the batch synthesis process as described herein.

## DESCRIPTION OF THE INVENTION

As used herein the term "polymer" means a homopolymer or a copolymer. A "homopolymer" is a polymer prepared from only one monomer, while a "copolymer" is a polymer obtained from at least two different monomers.

As used herein, the expression "A and/or B" means "A, or B, or A and B".

The invention also includes all possible combinations of the various embodiments disclosed, whether they are preferred or exemplary embodiments when they are not mutually exclusive. Furthermore, where ranges of values are indicated, the end-values are part of these ranges. The disclosure also includes all combinations between the end-values of these ranges. For example, ranges "1-20, preferably 5-15", imply disclosure of the ranges "1-5", "1-15", "5-20" and "15-20".

### Step 1)

The mixture M1 comprises:

(i) from 50 to 97.899% by weight of at least one $\text{alkyl}_1$ (meth)acrylate ester;
(ii) from 0,1 to 40% by weight of at least one alkanolamine;
(iii) from 0.001 to 10 % of at least one catalyst;
(iv) from 2 to 30 % by weight of at least one solvent;

based on the total weight of the mixture M1;

The mixture M1 is advantageously prepared under stirring.

The stirring can be made by any means known to the skilled person. We can mention, for example, mechanical stirring devices such as 45° three or four-bladed impellers, Rushton turbines, marine propellers, whether single or multiple, combined or not, or a circulation loop equipped with a centrifugal pump and/or rotor-stator homogenizer could be utilized.

The stirring speed is advantageously comprised between 10 and 1 000 rpm (rotation per minute), preferably between 50 and 800 rpm.

In particular embodiment, the stirring is made via a static mixer, which is a piping element comprising helices or baffles or any other obstacle designed to increase turbulence, placed on a recirculation loop for reagents, catalyst and recycled flows upstream of the reactor.

The quantity of $\text{alkyl}_1$ (meth)acrylate ester is comprised between 50 and 97.899% by weight based on the total weight of the mixture M1, preferably between 60 and 90% by weight, more preferably between 65 and 85% by weight.

The $\text{alkyl}_1$ (meth)acrylate ester may be linear, cyclic (substituted or not) or branched. Preferably the $\text{alkyl}_1$ (meth) acrylate ester is linear and the alkyl chain comprises advantageously from 1 to 10 carbons, preferably between 1 and 8, more preferably between 1 and 4. Even more preferably the $\text{alkyl}_1$ group is methyl or ethyl. Even more preferentially the $\text{alkyl}_1$ (meth)acrylate is methyl(meth)acrylate.

The quantity of alkanolamine is comprised between 0,1 and 40% by weight based on the total weight of the mixture M1, preferably between 2 and 30% by weight, more preferably between 5 and 25% by weight.

The alkanolamine corresponds to a chemical compound that contains both hydroxyl (-OH) and amine functional groups on an alkane backbone. The amine functional group may be a primary ($-NH_2$), secondary ($-NHR_1$), or tertiary ($-NR_1R_2$) amine. Preferably the amine functional group is a tertiary amine ($-NR_1R_2$). The alkane backbone is a hydrocarbon chain comprising between 1 and 10 carbon atoms.

The alkyl radicals of the tertiary amine -$R_1$ and -$R_2$ are hydrocarbon chains comprising, each independently, between 1 and 8 carbon atoms, preferably between 1 and 4 carbon atoms. More preferably -$R_1$ and -$R_2$ have each 1 carbon atom.

The quantity of catalyst is comprised between 0.001 and 10 % by weight based on the total weight of the mixture M1, preferably between 0,01 et 5% by weight.

The quantity of catalyst is advantageously comprised between 0.1 and 150 mmol for one mol of alkanolamine, preferably between 1 and 30 mmol, more preferably between 1.1 and 25 mmol by alkanolamine mol.

The catalyst is an organometallic derivative. Choosing the catalyst depends on various criteria, like the nature of the amino alkyl (meth)acrylate to be synthesized or the nature of the alcohol used. For example, the organometallic can be a derivate of tin, titanium, zirconium or zinc. Non-limiting list of catalysts are titanium tetra isopropoxyde (TTIP), zirconium tetraacetylacetonate. Preferably the catalyst is a derivate of tin, more preferably a dialkyltin oxide. Even more preferably the catalyst is dibutyltin oxide (DBTO).

The quantity of the different compounds constituting the mixture M1 are adjusted so as not to exceed 100% by weight.

In a preferred embodiment, the catalyst is in the form of a suspension S1 in which 70% to 95% by weight of the catalyst is solubilized, and 5% to 30% by weight of catalyst is in the form of particles suspended in the suspension S1 based on the total amount of catalyst in the suspension S1. Preferably, more than 80% and no more than 95%, more preferably more than 85% and no more than 95% by weight of catalyst is solubilized in the suspension S1.

The molar ratio between the alkyl$_1$ (meth)acrylate ester and the total amount of alkanolamine used in the batch synthesis process (step 1 + step 2) is advantageously comprised between 1:5 and 5:1, preferably between 1:2 and 2:1.

The solvent used in the process of the invention is advantageously an inert solvent, i.e. a solvent that does not react with the alkanolamine and the alkyl$_1$ (meth)acrylate ester, advantageously selected from linear, branched or cyclic hydrocarbons, saturated or unsaturated, containing from 3 to 16 carbons. Preferably, the solvent is selected from n-hexane, the isomers of n-hexane, and mixtures thereof.

The quantity of solvent is comprised between 2 and 30% by weight based on the total weight of the mixture M1, preferably between 3 and 20% by weight, more preferably between 4 and 15% by weight.

**Step 2)**

In a second step, the mixture M1 is heated at a temperature T1 comprised between 40°C and 120°C and the alkyl$_1$ alcohol that forms during the reaction is removed by azeotropic distillation. The azeotrope is formed between the solvent and the alkyl$_1$ alcohol resulting from the transesterification of the alkyl$_1$ (meth) acrylate ester with the alkanolamine. By distilling the mixture M1, the thermodynamic equilibrium is shifted toward the formation of the amino alkyl (meth)acrylates. The distillation time is comprised between 30 minutes and 720 minutes. During the distillation, an additional quantity of alkanolamine is further added continuously.

The mixture M1 is heated at a temperature T1 comprised between 40°C and 120°C, preferably between 70 and 100°C.

The azeotropic distillation lasts advantageously between 30 minutes and 720 minutes, preferably between 240 minutes and 500 minutes.

The additional quantity of the alkanolamine added continuously is comprised between 15 and 85% by weight based on the total amount of alkanolamine used in steps 1) and 2), preferably between 20 and 85%, more preferably between 40 and 85%, more preferably between 50 and 85%, more preferably between 60 and 85%, more preferably between 20 and 80% by weight, more preferably between 40 and 80%, more preferably between 50 and 80%, more preferably between 60 and 80%, more preferably between 20 and 75%, more preferably between 40 and 75%, more preferably between 50 and 75% and even more preferably between 60 and 75% by weight.

The rate of addition of the at least alkanolamine advantageously corresponds to the evaporation rate of the alkyl$_1$ alcohol.

In step 2)(ii), the alkanolamine is continuously added while the alkyl$_1$ alcohol evaporates. The addition lasts advantageously between 30 to 720 min.

In other terms, in step 2)(ii), the alkanolamine is added while the alkyl$_1$ alcohol is simultaneously distilled off.

In a preferred embodiment, the addition of the alkanolamine is divided in three phases:

(i) first phase: addition of between 10 and 40% by weight of the total alkanolamine continuously added in step 2 for between 10 and 40% of the total distillation time of step 2);
(ii) second phase : addition of between 20 and 60% by weight of the total alkanolamine continuously added in step 2 for between 20% and 50% of the total distillation time of step 2);
(iii) third phase : addition of between 10 and 25% by weight of the total alkanolamine continuously added in step 2 for between 10 and 30% of the total distillation time of step 2).

In a particularly preferred embodiment, the addition of the alkanolamine in step 2) is carried out in three phases:

(i) first phase: addition of between 10 and 40% by weight of the total amount of alkanolamine (step 1 + step 2), this addition being carried out continuously over a period representing 10% to 40% of the total addition time of alkanolamine in step 2;
(ii) second phase : addition of between 20 and 60% by weight of the total amount of alkanolamine(step 1 + step 2), this addition being carried out continuously over a period representing 20% to 50% of the total addition time of alkanolamine in step 2;
(iii) third phase : addition of between 10 and 25% by weight of the total amount of alkanolamine (step 1 + step 2), this addition being carried out continuously over a period representing 10% to 30% of the total addition time of alkanolamine in step 2.

The sum of the addition time of the three phases (i) to (iii) is equal to the total addition time of alkanolamine in step 2 (addition time of phase (i) + phase (ii) + phase (iii) = 100% of the addition time of alkanolamine in step 2).
The sum of the amount of alkanolamine added between the three phases will be adjusted by the skilled person as that it not exceed 85% by weight based on the total amount of alkanolamine used in steps 1) and 2),.
Advantageously, in this preferred embodiment, the rate of addition of the alkanolamine will be adjusted by the skilled person so that the rate of addition of the alkanolamine in the second phase (ii) is higher than in the first phase (i) (second phase (ii) > first phase (i)) and the rate of addition of the alkanolamine in the third phase (iii) is slower than in the second phase (ii) (third phase (iii) < second phase (ii)).
In a particular embodiment, an additional quantity of catalyst is added continuously in step 2)(ii).
The additional quantity of catalyst added continuously is advantageously comprised between 1 and 80% by weight based on the total weight of catalyst used in steps 1) and 2), preferably between 30 and 50% by weight.
The additional catalyst can be added in solid form or as a suspension (suspension S1 as defined above) or in liquid form.
The addition of the alkanolamine in step 2)(ii) is advantageously initiated when the mixture M1 reaches its boiling point.
Preferably the addition of the alkanolamine, in step 2)(ii), is initiated when the alkyl$_1$ alcohol starts evaporating.

**Step 3)**

In step 3) the amino alkyl (meth)acrylate is recovered by distillation of the mixture M2.
The distillation can be carried out at atmospheric pressure, or at a pressure below the atmospheric pressure. Preferably the distillation is carried out at below the atmospheric pressure.
When the distillation is carried out at a pressure below the atmospheric pressure, the pressure is advantageously comprised between 1 and less than 1000 mbar absolute (1 mbar = 100 Pa). The pressure is preferably less than 900 mbar absolute, more preferably less than 800 mbar absolute, more preferably less than 700 mbar absolute, more preferably less than 600 mbar absolute, more preferably less than 500 mbar absolute, more preferably less than 400 mbar absolute, more preferably less than 300 mbar absolute, more preferably less than 200 mbar absolute, more preferably less than 100 mbar absolute and even more preferably less than 50 mbar absolute, and advantageously more than 1 mbar absolute. Absolute pressure corresponds to pressure relative to zero pressure (vacuum).
The distillation time is advantageously comprised between 30 minutes and 600 minutes, preferably between 60 minutes and 360 minutes.
To accelerate the distillation step, the mixture M2 can be heated, advantageously between 60 and 120 °C, preferably between 80 and 100°C.

**Optional step(s))**

The batch synthesis process according to the present disclosure can comprise additional optional step(s). For example, after its recovery, the amino alkyl (meth)acrylate can be purified, for example by distillation.
When using an organotin catalyst, the latter can be recycled as described in patent application WO2019196048 filed by the applicant, or it can be recycled as described in patent application CN117000313, of the applicant.
In a preferred embodiment, the steps 1 to 3 of the batch process are repeated.
In this embodiment, the process advantageously involves recycling the catalyst.
In a particular embodiment, at least one of the alkyl$_1$ (meth)acrylate ester, the alkanolamine and the solvent, of the batch synthesis process, originates from a feedstock comprising recycled material or materials of biological origin.

Another object of the invention relates to the amino alkyl (meth)acrylate obtained from the batch synthesis process according to the invention.

Another object of the present disclosure concerns a polymer obtained from the polymerization of at least one amino alkyl (meth)acrylate obtained from the batch synthesis as described herein.

The invention and the advantages thereof will be better illustrated by, but not limited to, the following examples given to illustrate the invention.

**List of abbreviations:**

DMAEA: 2-dimethylaminoethyl acrylate
MA: Methylacrylate
DMOH: Dimethylaminoethanol
Hx: Hexane
DBTO: Dibutyl tin oxide
Ptz: phenothiazine
GC-FID: Gas chromatography flame ionization detector
MeOH: Methanol
ICP-OES: Inductively coupled plasma - optical emission spectroscopy

**EXAMPLES**

**Example 1: Batch synthesis of DMAEA - General procedure**

a) Preparation of the mixture M1 (step 1)

In a 250 mL three-neck round-bottom flask, the MA, DMOH, Hx, DBTO and Ptz are added to form a mixture M1. The reaction mixture M1 is stirred at 500 rpm.

A 50 cm-height column, 20 mm inner diameter packed with Dixon rings (3 mm) is mounted, on the central neck, and a cooling condenser containing glycol water at -10°C is placed on the top of the column.

A heated plate is used to heat the mixture M1 at 110°C.

b) Preparation of the mixture M2 (step 2)

A certain amount of DMOH is then continuously added in the reaction mixture using a metering pump for 4 h.

The distillate is consistently collected at the upper section of the column by regulating the temperature within the range of 48 to 53°C at the column's top. This temperature range indicated that the vapor composition is close to the azeotrope mixture Hx/MeOH.

A reflux rate control valve is used to collect the distillate, control the reflux rate and indirectly adjust the temperature of heating.

For every 10 mL of distillate collected, 7 mL of Hx is reintroduced into the reacting medium to mimic an industrial process using a Hx/MeOH phase separation and Hx reinjection.

The reaction continues until the temperature at the top of the column cannot be maintained below 53°C, indicating that the reaction is complete.

The reaction mixture is analysed with a GC-FID method.

A GC Agilent 7820A is used with a DB-WAX UI column of 30m and $0.50\mu m$ as inner diameter.

A flame ionisation detector is used for the detection.

5 sample standards with different compositions are injected with DMAEA, DMOH, MA, MeOH, Hx to establish the calibration curves.

The obtained response factor for DMAEA is applied to quantify the heavier components observed after a retention time of 16 minutes.

The DMAEA conversion rate is calculated as

$$\mathrm{DMAEA}\,convertion\,rate(\%) = \left(\frac{\%\mathrm{DMAEA}}{(\%\mathrm{DMAEA} + \%\mathrm{DMOH} + \%\mathrm{Hv})}\right) \mathrm{x}\,100$$

c) Recovering of DMAEA (step 3)

The reaction mixture is then distilled under heating and vacuum pressure, using the same apparatus as described above. DMAEA is collected when the upper column temperature reaches 78°C at 40 mbar down to 5 mbar absolute. 90% of the DMAEA contained in the mixture is recovered by distillation. The DMAEA fraction is weighted and analysed using the GC-FID method. The lower residue is collected and weighed.

Example 1 (**E1**) is carried out according to the invention with a quantity of DMOH added in step 2)(ii) of 75 % by weight.

Example 1 (**E2**) is carried out according to the invention with a quantity of DMOH added in step 2)(ii) of 50 % by weight

Example 1 (**E3**) is carried out according to the invention with a quantity of DMOH added in step 2)(ii) of 85 % by weight

Example 1 (**E4**) is carried out according to the invention with a quantity of DMOH added in step 2)(ii) of 20 % by weight.

Comparative examples 2 to 4 and 6 (**CE2** to **CE4** and **CE6**) are carried out using quantities of DMOH in step 2)(ii) out of the scope of the invention.

Comparative examples 5 (**CE5**) is carried as Example 1 but the additional quantity of DMOH in step 2) is added in one shot and not continuously through the distillation.

The compositions of steps 1) and 2) in the different examples are presented in Table 1.

Table 1. Quantities of raw materials used in steps 1 and 2 of the batch process of the different compositions (**E1** to **E4** according to the invention and comparative examples **CE2** to **CE6**). * DMOH added in one shot and not in continuous through the azeotropic distillation.

| | | | | | DMOH | |
|---|---|---|---|---|---|---|
| **Examples** | **MA (g)** | **Hx (g)** | **Catalsyt DBTO (g)** | **Ptz (g)** | **Step 1 (g) (wt% relative to the total quantity of DMOH in steps 1+2)** | **Step 2 (g) (wt% relative to the total quantity of DMOH in steps 1+2)** |
| **E1** | 89.0 | 13.0 | 1.8 | 0.1 | 11.5 (25 wt%) | 34.5 (75 wt%) |
| **E2** | 89.0 | 13.0 | 1.8 | 0.1 | 23 (50 wt%) | 23 (50 wt%) |
| **E3** | 89.0 | 13.0 | 1.8 | 0.1 | 6.9 (15 wt%) | 39.1 (85 wt%) |
| **E4** | 89.0 | 13.0 | 1.8 | 0.1 | 37 (80 wt%) | 9 (20 wt%) |
| **CE2** | 89.0 | 13.0 | 1.8 | 0.1 | 46 (100 wt%) | 0 |
| **CE3** | 89.0 | 13.0 | 1.8 | 0.1 | 4.6 (10 wt%) | 41.4 (90 wt%) |
| **CE4** | 89.0 | 13.0 | 1.8 | 0.1 | 0 | 46 (100 wt%) |
| **CE5** | 89.0 | 13.0 | 1.8 | 0.1 | 11.5 (25 wt%) | 34.5* (75 wt%) |
| **CE6** | 89.0 | 13.0 | 1.8 | 0.1 | 41.4 (90 wt%) | 4.6 (10 wt%) |

After distillation of the DMAEA product, the lower residue is collected and quantity of initial raw materials are adjusted with fresh ones to be used in a second DMAEA reaction.

The batch process is repeated 4 times (hereafter referred with numbers x.1 to x.4) in the same reaction conditions than for the first reaction (hereafter referred as "x.0").

The quantities of DMAEA, residual DMOH, heavies, waste and the conversion and reaction time of each reaction process is assessed.

The results are presented below in Tables 2 to 9.

*1) Results of a batch process according to the invention with 75 wt% of DMOH added in step 2) (E1.0 to E1.4)*

Table 2. Results of the 5 DMAEA batch reaction process synthesis carried out according to the invention adding 75 wt% of DMOH in step 2) (**E1.0** to **E1.4**).

| Example 1 | E1.0 | E1.1 | E1.2 | E1.3 | E1.4 |
|---|---|---|---|---|---|
| DMAEA (wt%) | 69.8 | 68.9 | 67.6 | 67.5 | 67.4 |
| DMOH (wt%) | 0.5 | 0.6 | 0.6 | 0.5 | 0.5 |
| Heavies (wt%) | 0.1 | 0.3 | 0.4 | 0.5 | 0.6 |
| Reaction time (h) | 6.5 | 6.6 | 6.7 | 6.5 | 6.7 |
| Conversion (%) | 99.1 | 98.7 | 98.6 | 98.5 | 98.3 |
| Final waste (g) | 5.8 | 6.1 | 6.1 | 6.2 | 6.4 |
| Waste/ DMAEA (%wt/wt) | 0.062 | 0.065 | 0.067 | 0.068 | 0.070 |

*2) Results of a batch process according to the invention with 50 wt% of DMOH added in step 2) (E2.0 to E2.4)*

Table 2. Results of the 5 DMAEA batch reaction process synthesis carried out according to the invention adding 50 wt% of DMOH in step 2) (**E2.0** to **E2.4**).

| Example 2 | E2.0 | E2.1 | E2.2 | E2.3 | E2.4 |
|---|---|---|---|---|---|
| DMAEA (wt%) | 68.5 | 68.2 | 66.5 | 66.7 | 69.1 |
| DMOH (wt%) | 1.2 | 1.5 | 1.0 | 1.6 | 1.1 |
| Heavies (wt%) | 0.3 | 0.7 | 0.9 | 1.1 | 1.3 |
| Reaction time (h) | 7 | 7.5 | 7.7 | 7.2 | 7.1 |
| Conversion (%) | 97.9 | 96.9 | 97.2 | 96.1 | 96.6 |
| Final waste (g) | 6.5 | 7.3 | 7.2 | 7.9 | 8.0 |
| Waste/ DMAEA (%wt/wt) | 0.070 | 0.079 | 0.080 | 0.089 | 0.086 |

*3) Results of a batch process according to the invention with 85 wt% of DMOH added in step 2) (E3.0 to E3.4)*

Table 3. Results of the 5 DMAEA batch reaction process synthesis carried out according to the invention adding 85 wt% of DMOH in step 2) (**E3.0** to **E3.4**).

| Example 3 | E3.0 | E3.1 | E3.2 | E3.3 | E3.4 |
|---|---|---|---|---|---|
| DMAEA (wt%) | 68.5 | 69.2 | 67.6 | 68.4 | 68.8 |
| DMOH (wt%) | 1.2 | 1.5 | 1.0 | 1.6 | 1.1 |
| Heavies (wt%) | 0.2 | 0.5 | 0.7 | 0.7 | 0.8 |
| Reaction time (h) | 6.3 | 6.2 | 6.9 | 6 | 6.2 |
| Conversion (%) | 98.0 | 97.2 | 97.6 | 96.7 | 97.4 |
| Final waste (g) | 6.4 | 7.1 | 6.8 | 7.4 | 7.1 |
| Waste/ DMAEA (%wt/wt) | 0.069 | 0.076 | 0.075 | 0.080 | 0.077 |

*4) Results of a batch process according to the invention with 20 wt% of DMOH added in step 2) (E4.0 to E4.4)*

Table 4. Results of the 5 DMAEA batch reaction process synthesis carried out according to the invention adding 20 wt% of DMOH in step 2) (E4.0 to E4.4).

| Example 4 | E4.0 | E4.1 | E4.2 | E4.3 | E4.4 |
|---|---|---|---|---|---|
| DMAEA (wt%) | 68,2 | 69,5 | 68,5 | 68,7 | 68,8 |
| DMOH (wt%) | 1.2 | 1.1 | 1.3 | 1.5 | 0.9 |
| Heavies (wt%) | 0.3 | 0.7 | 1.0 | 1.2 | 1.6 |
| Reaction time (h) | 6.3 | 6.5 | 6.6 | 5.8 | 6.2 |
| Conversion (%) | 97.8 | 97.5 | 96.8 | 96.2 | 96.5 |
| Final waste (g) | 6.5 | 7.0 | 7.6 | 8.1 | 8.3 |
| Waste/ DMAEA (%wt/wt) | 0.070 | 0.075 | 0.082 | 0.087 | 0.089 |

5) Results of a comparative batch process wherein no DMOH is added in step 2) (CE2.0 to CE2.4)

Table 5. Results of the 5 DMAEA batch reaction process synthesis with no addition of DMOH in step 2) (CE2.0 to CE2.4).

| Comparative Example 2 (CE2) | CE2.0 | CE2.1 | CE2.2 | CE2.3 | CE2.4 |
|---|---|---|---|---|---|
| DMAEA (wt%) | 69.1 | 68.1 | 67.1 | 64.1 | 62.0 |
| DMOH (wt%) | 0.5 | 1.0 | 1.5 | 5.1 | 6.0 |
| Heavies (wt%) | 0.4 | 0.7 | 1.1 | 1.4 | 1.7 |
| Reaction time (h) | 7 | 7.5 | 8 | 8.5 | 9 |
| Conversion (%) | 98.8 | 97.6 | 96.3 | 90.8 | 88.9 |
| Final waste (g) | 6.1 | 6.9 | 7.8 | 10.7 | 11.8 |
| Waste/ DMAEA (%wt/wt) | 0.07 | 0.07 | 0.09 | 0.12 | 0.14 |

6) Results of a comparative batch process with 90 wt% of DMOH added in step 2) (CE3.0 to CE3.4)

Table 6. Results of the 5 DMAEA batch reaction process synthesis adding 90 wt% of DMOH in step 2) (CE3.0 to CE3.4).

| Comparative Examples 3 (CE3) | CE3.0 | CE3.1 | CE3.2 | CE3.3 | CE3.4 |
|---|---|---|---|---|---|
| DMAEA (wt%) | 68.7 | 68.1 | 67.1 | 65.1 | 64.1 |
| DMOH (wt%) | 0.5 | 0.8 | 1.0 | 2.8 | 3.3 |
| Heavies (wt%) | 0.2 | 0.5 | 0.7 | 1.0 | 1.2 |
| Reaction time (h) | 9 | 7 | 8.5 | 9 | 9 |
| Conversion (%) | 98.9 | 98.1 | 97.4 | 94.5 | 93.5 |
| Final waste (g) | 5.9 | 6.5 | 6.9 | 8.4 | 9.0 |
| Waste/ DMAEA (%wt/wt) | 0.057 | 0.063 | 0.069 | 0.086 | 0.094 |

7) Results of a comparative batch process with 100 wt% of DMOH added in step 2) (CE4.0 to CE4.4)

Table 7. Results of the 5 DMAEA batch reaction process synthesis adding 100 wt% of DMOH in step 2) (**CE4.0** to **CE4.4**).

| Comparative Examples 4 (CE4) | CE4.0 | CE4.1 | CE4.2 | CE4.3 | CE4.4 |
|---|---|---|---|---|---|
| DMAEA (wt%) | 68.9 | 68.1 | 67.1 | 64.6 | 63.1 |
| DMOH (wt%) | 0.5 | 0.9 | 1.3 | 4.0 | 4.6 |
| Heavies (wt%) | 0.3 | 0.6 | 0.9 | 1.2 | 1.5 |
| Reaction time (h) | 10 | 9 | 8.5 | 9 | 9 |
| Conversion (%) | 98.8 | 97.9 | 96.9 | 92.6 | 91.2 |
| Final waste (g) | 6.0 | 6.7 | 7.3 | 9.6 | 10.4 |
| Waste/ DMAEA (%wt/wt) | 0.058 | 0.065 | 0.073 | 0.099 | 0.110 |

*8) Results of a comparative batch process with 85 wt% of DMOH added in one shot in step 2) (CE5.0 to CE5.4)*

Table 8. Results of the 5 DMAEA batch reaction process synthesis adding DMOH in step 2) in one shot (**CE5.0** to **CE5.4**).

| Comparative Example 5 (CE5) | CE5.0 | CE5.1 | CE5.2 | CE5.3 | CE5.4 |
|---|---|---|---|---|---|
| DMAEA (wt%) | 68.9 | 68.1 | 66.7 | 64.5 | 63.0 |
| DMOH (wt%) | 0.6 | 1.1 | 1.4 | 4.5 | 5.8 |
| Heavies (wt%) | 0.4 | 0.7 | 1.1 | 1.4 | 1.7 |
| Reaction time (h) | 7.1 | 7.8 | 7.9 | 8.4 | 8.8 |
| Conversion (%) | 98.7 | 97.5 | 96.4 | 916 | 89.3 |
| Final waste (g) | 6.1 | 6.9 | 7.7 | 103 | 11.7 |
| Waste/ DMAEA (%wt/wt) | 0.066 | 0.075 | 0.085 | 0.119 | 0.138 |

*9) Results of a comparative batch process with 10 wt% of DMOH added in step 2) (CE6.0 to CE6.4)*

Table 9. Results of the 5 DMAEA batch reaction process synthesis adding 10 wt% of DMOH in step 2) (**CE6.0** to **CE6.4**).

| Comparative Example 6 (CE6) | CE6.0 | CE6.1 | CE6.2 | CE6.3 | CE6.4 |
|---|---|---|---|---|---|
| DMAEA (wt%) | 69.1 | 67.5 | 65.0 | 63.5 | 64.2 |
| DMOH (wt%) | 0.5 | 1.0 | 1.5 | 5.1 | 6.0 |
| Heavies (wt%) | 0.5 | 0.9 | 1.4 | 1.8 | 2.2 |
| Reaction time (h) | 7.5h | 7.7h | 8 h | 8.8h | 10h |
| Conversion (%) | 98.6 | 97.3 | 95.8 | 90.2 | 88.6 |
| Final waste (g) | 6.3 | 7.1 | 8.1 | 11.3 | 12.7 |
| Waste/ DMAEA (%wt/wt) | 0.060 | 0.071 | 0.083 | 0.119 | 0.132 |

The results demonstrate that the addition of a specific amount of DMOH in continuous in step 2) according to the invention allows maintaining good conversions and yield of DMAEA when the reaction is carried out in batch process and also leads to reduced amounts of waste and heavies.

From theses tables it can be seen that when a specific amount of DMOH is added at the charge of the synthesis of amino alkyl (meth)acrylates and a specific amount is added continuously during the synthesis, the synthesis is improved, regarding the yield, the recyclability of the catalyst, a reduction of the waste as less heavies are formed and in term of synthesis kinetic.

**Batch synthesis of DMAEA with the addition of DMOH in step 2) in three phases**

Example 1 is reproduced except that the amount of DMOH added in step 2) (34.5 g, 75wt% based on the total amount of DMOH added between steps 1+2) is added in 3 phases (phase I, II and III). In each phase, a specific amount of DMOH (based on the total amount of DMOH (step 1 + step 2)) is added in a specific duration based on the total addition time of DMOH added in step 2) of 4 hours.

The sum of addition time of the 3 phases (I, II and III) equals to the total addition time of the casting of DMOH in step 2) (that is 4 hours = 100%).

The amount of DMOH added and the duration of each phase are summarized in table 10.

Table 10 - Composition of the three phases of addition of DMOH of step 2 in the DMAEA batch process.

| Examples | Phase I | | Phase II | | Phase III | |
|---|---|---|---|---|---|---|
| | Amount of DMOH (%)* | Addition time (%)** | Amount of DMOH (%)* | Addition time (%)** | Amount of DMOH (%)* | Addition time (%)** |
| E5 | 18 | 29 | 35 | 43 | 22 | 28 |
| E6 | 15 | 35 | 46 | 44 | 14 | 21 |
| E7 | 12 | 39 | 45 | 31 | 18 | 30 |
| E8 | 9 | 30 | 46 | 40 | 20 | 30 |
| E9 | 41 | 30 | 19 | 40 | 15 | 30 |
| E10 | 20 | 41 | 40 | 29 | 15 | 30 |
| E11 | 31 | 30 | 19 | 40 | 25 | 30 |
| E12 | 20 | 24 | 40 | 51 | 15 | 25 |
| E13 | 20 | 30 | 46 | 40 | 9 | 30 |
| E14 | 20 | 30 | 29 | 40 | 26 | 30 |
| E15 | 20 | 34 | 40 | 35 | 15 | 31 |
| *based on the total amount of DMOH added in steps 1+2 **based on the total addition time of DMOH added in step 2 | | | | | | |

*Results*

After distillation of the DMAEA product, the lower residue is collected and quantity of initial raw materials are adjusted with fresh ones to be used in a second DMAEA batch process reaction.

The batch process is repeated 2 times (hereafter referred with numbers x.1 to x.2) in the same reaction conditions than for the first reaction (hereafter referred as "x.0").

The quantities of DMAEA, residual DMOH, heavies, waste and the conversion and reaction time of each reaction process is assessed for each example E5 to E15.

The results are presented in Tables 11 to 21.

Table 11 - Results of the 3 DMAEA batch reaction process with the addition of DMOH in step 2) carried out in three phases according to example 5.

| Example 5 | E5.0 | E5.1 | E5.2 |
|---|---|---|---|
| DMAEA (wt%) | 70.1 | 70.0 | 69.7 |
| DMOH (wt%) | 0.3 | 0.5 | 0.5 |
| Heavies (wt%) | 0.1 | 0.2 | 0.3 |
| Reaction time (h) | 6 | 6.2 | 6.4 |
| Conversion (%) | 99.4 | 99.1 | 98.9 |

(continued)

| Example 5 | E5.0 | E5.1 | E5.2 |
|---|---|---|---|
| Final waste (g) | 5.7 | 5.8 | 6.0 |
| Waste/ DMAEA (%wt/wt) | 0.060 | 0.061 | 0.064 |

Table 12 - Results of the 3 DMAEA batch reaction process with the addition of DMOH in step 2) carried out in three phases according to example 6.

| Example 6 | E6.0 | E6.1 | E6.2 |
|---|---|---|---|
| DMAEA (wt%) | 69.9 | 69.7 | 69.6 |
| DMOH (wt%) | 0.4 | 0.5 | 0.6 |
| Heavies (wt%) | 0.1 | 0.2 | 0.2 |
| Reaction time (h) | 6.1 | 6.2 | 6.4 |
| Conversion (%) | 99.3 | 99.1 | 98.8 |
| Final waste (g) | 5.7 | 5.8 | 6.1 |
| Waste/ DMAEA (%wt/wt) | 0.061 | 0.062 | 0.065 |

Table 13 - Results of the 3 DMAEA batch reaction process with the addition of DMOH in step 2) carried out in three phases according to example 7.

| Example 7 | E7.0 | E7.1 | E7.2 |
|---|---|---|---|
| DMAEA (wt%) | 69.7 | 69.6 | 69.4 |
| DMOH (wt%) | 0.4 | 0.5 | 0.7 |
| Heavies (wt%) | 0.1 | 0.2 | 0.3 |
| Reaction time (h) | 6.2 | 6.3 | 6.5 |
| Conversion (%) | 99.2 | 99.0 | 98.6 |
| Final waste (g) | 5.7 | 5.8 | 6.2 |
| Waste/ DMAEA (%wt/wt) | 0.061 | 0.062 | 0.066 |

Table 14 - Results of the 3 DMAEA batch reaction process with the addition of DMOH in step 2) carried out in three phases according to according to example 8.

| Example 8 | E8.0 | E8.1 | E8.2 |
|---|---|---|---|
| DMAEA (wt%) | 68.6 | 68.2 | 68.1 |
| DMOH (wt%) | 1.1 | 1.3 | 1.5 |
| Heavies (wt%) | 0.2 | 0.3 | 0.4 |
| Reaction time (h) | 6.1 | 6.5 | 6.4 |
| Conversion (%) | 98.1 | 97.7 | 97.3 |
| Final waste (g) | 6.3 | 6.6 | 6.8 |
| Waste/ DMAEA (%wt/wt) | 0.068 | 0.071 | 0.075 |

Table 15 - Results of the 3 DMAEA batch reaction process with the addition of DMOH in step 2) carried out in three phases according to according to example 9.

| Example 9 | E9.0 | E9.1 | E9.2 |
|---|---|---|---|
| DMAEA (wt%) | 68.2 | 68.3 | 67.9 |
| DMOH (wt%) | 1.2 | 1.4 | 1.5 |

(continued)

| Example 9 | E9.0 | E9.1 | E9.2 |
|---|---|---|---|
| Heavies (wt%) | 0.2 | 0.3 | 0.5 |
| Reaction time (h) | 6.2 | 6.3 | 6.5 |
| Conversion (%) | 98.0 | 97.6 | 97.1 |
| Final waste (g) | 6.3 | 6.6 | 7.0 |
| Waste/ DMAEA (%wt/wt) | 0.069 | 0.072 | 0.076 |

Table 16 - Result of the 3 DMAEA batch reaction process with the addition of DMOH in step 2) carried out in three phases according to according to example 10.

| Example 10 | E10.0 | E10.1 | E10.2 |
|---|---|---|---|
| DMAEA (wt%) | 68.5 | 68.4 | 68.0 |
| DMOH (wt%) | 1.1 | 1.3 | 1.7 |
| Heavies (wt%) | 0.1 | 0.3 | 0.3 |
| Reaction time (h) | 6 | 6.2 | 6.3 |
| Conversion (%) | 98.3 | 97.7 | 96.9 |
| Final waste (g) | 6.1 | 6.6 | 7.1 |
| Waste/ DMAEA (%wt/wt) | 0.066 | 0.071 | 0.078 |

Table 17 - Results of the 3 DMAEA batch reaction process with the addition of DMOH in step 2) carried out in three phases according to according to example 11.

| Example 11 | E11.0 | E11.1 | E11.2 |
|---|---|---|---|
| DMAEA (wt%) | 68.8 | 68.6 | 67.9 |
| DMOH (wt%) | 1.3 | 1.5 | 1.6 |
| Heavies (wt%) | 0.3 | 0.4 | 0.5 |
| Reaction time (h) | 6.3 | 6.5 | 6.9 |
| Conversion (%) | 97.7 | 97.3 | 97.0 |
| Final waste (g) | 6.6 | 6.9 | 7.1 |
| Waste/ DMAEA (%wt/wt) | 0.071 | 0.074 | 0.077 |

Table 18 - Results of the 3 DMAEA batch reaction process with the addition of DMOH in step 2) carried out in three phases according to according to example 12.

| Example 12 | E12.0 | E12.1 | E12.2 |
|---|---|---|---|
| DMAEA (wt%) | 68.8 | 68.1 | 67.5 |
| DMOH (wt%) | 1.1 | 1.1 | 1.4 |
| Heavies (wt%) | 0.2 | 0.4 | 0.6 |
| Reaction time (h) | 6.2 | 6.4 | 6.8 |
| Conversion (%) | 98.7 | 97.8 | 97.1 |
| Final waste (g) | 6.3 | 6.5 | 7 |
| Waste/ DMAEA (%wt/wt) | 0.068 | 0.071 | 0.077 |

Table 19 - Results of the 3 DMAEA batch reaction process with the addition of DMOH in step 2) carried out in three phases according to according to example 13.

| Example 13 | E13.0 | E13.1 | E13.2 |
|---|---|---|---|
| DMAEA (wt%) | 68.1 | 67.8 | 67.5 |
| DMOH (wt%) | 1.4 | 1.5 | 1.7 |
| Heavies (wt%) | 0.2 | 0.4 | 0.5 |
| Reaction time (h) | 6.1 | 6.5 | 6.3 |
| Conversion (%) | 97.7 | 97.3 | 96.8 |
| Final waste (g) | 6.5 | 6.8 | 7.1 |
| Waste/ DMAEA (%wt/wt) | 0.070 | 0.075 | 0.078 |

Table 20 - Results of the 3 DMAEA batch reaction process with the addition of DMOH in step 2) carried out in three phases according to according to example 14.

| Example 14 | E14.0 | E14.1 | E14.2 |
|---|---|---|---|
| DMAEA (wt%) | 68.9 | 68.2 | 68.4 |
| DMOH (wt%) | 1.3 | 1.7 | 1.6 |
| Heavies (wt%) | 0.2 | 0.4 | 0.7 |
| Reaction time (h) | 6.3 | 6.2 | 6.4 |
| Conversion (%) | 97.9 | 97 | 96.7 |
| Final waste (g) | 6.5 | 7.0 | 7.4 |
| Waste/ DMAEA (%wt/wt) | 0.069 | 0.076 | 0.080 |

Table 21 - Results of the 3 DMAEA batch reaction process with the addition of DMOH in step 2) carried out in three phases according to according to example 15.

| Example 15 | E15.0 | E15.1 | E15.2 |
|---|---|---|---|
| DMAEA (wt%) | 68.7 | 68.7 | 68.2 |
| DMOH (wt%) | 0.9 | 1.3 | 1.9 |
| Heavies (wt%) | 0.5 | 0.4 | 0.7 |
| Reaction time (h) | 6.1 | 6.2 | 6.6 |
| Conversion (%) | 98.1 | 97.4 | 96.3 |
| Final waste (g) | 6.4 | 6.9 | 7.6 |
| Waste/ DMAEA (%wt/wt) | 0.070 | 0.074 | 0.083 |

**Claims**

1.  Batch synthesis process of an amino alkyl (meth)acrylate comprising the following successive steps:

    1) Preparation of a mixture M1 comprising:

        (i) from 50 to 97.899% by weight of at least one $alkyl_1$ (meth)acrylate ester;
        (ii) from 0.1 to 40% by weight of at least one alkanolamine;
        (iii) from 0.001 and 10 % by weight of at least one organometallic catalyst;
        (iv) from 2 to 30 % by weight of at least one solvent;

    based on the total weight of the mixture M1;

2) preparation of a mixture M2 by:

(i) Heating the mixture M1 to a temperature T1 comprised between 40°C and 120°C and removing the $alkyl_1$ alcohol that forms during the reaction by azeotropic distillation; the azeotropic distillation is carried out between 30 to 720 min at T1,
(ii) further addition in continuous, during the azeotropic distillation, of a quantity of the at least one alkanolamine comprised between 15 and 85% by weight based on the total amount of alkanolamine used in steps 1) and 2);

3) recovering the amino alkyl (meth)acrylate by distillation of the mixture M2,

the amine of the alkanolamine is a tertiary amine $-NR_1R_2$ wherein $R_1$ and $R_2$, identical or different, are alkyl groups comprising between 1 and 8 carbon atoms and
the $alkyl_1$ (meth)acrylate ester is linear and the $alkyl_1$ chain comprises between 1 to 10 carbons.

2. Batch synthesis process according to claim 1, wherein the $alkyl_1$ (meth)acrylate ester is methyl(meth)acrylate.

3. Batch synthesis process according to any one of claims 1 to 2, wherein the alkanolamine is dimethylaminoethanol.

4. Batch synthesis process according to any one of claims 1 to 3, wherein the catalyst is in the form of a suspension S1 in which 70% to 95% by weight of the catalyst is solubilized, and 5% to 30% by weight of the catalyst is in the form of particles suspended in the suspension S1, based on the total amount of catalyst in the suspension S1.

5. Batch synthesis process according to any one of claims 1 to 4, wherein the quantity of alkanolamine added in step 2)(ii) is comprised between 20 and 80% by weight based on the total weight of alkanolamine used in steps 1) and 2).

6. Batch synthesis process according to any one of claims 1 to 5, wherein in step 2)(ii), the alkanolamine is continuously added while the $alkyl_1$ alcohol evaporates, and the addition lasts between 30 to 720 min.

7. Batch synthesis process according to any one of claims 1 to 6, wherein an additional quantity of catalyst is added continuously in step 2)(ii).

8. Batch synthesis process according to claim 7, wherein the catalyst is added continuously in an amount comprised between 1 and 80% by weight based on the total weight of catalyst used in steps 1) and 2).

9. Batch synthesis process according to any one of claims 1 to 8, wherein step 2)(ii) is initiated when the mixture M1 reaches its boiling point.

10. Batch synthesis process according to any one of claims 1 to 9, wherein step 2)(ii) is initiated when the $alkyl_1$ alcohol starts evaporating.

11. Batch synthesis process according to any one of claims 1 to 10, wherein steps 1 to 3 are repeated.

12. Batch synthesis process according to any one of claims 1 to 10, wherein steps 1 to 3 are repeated and wherein the batch synthesis process involves recycling the catalyst.

13. Batch synthesis process according to any one of claims 1 to 12, wherein at least one of the $alkyl_1$ (meth)acrylate ester, the alkanolamine and the solvent originates from a feedstock comprising recycled material or materials of biological origin.

**Patentansprüche**

1. Batch-Syntheseverfahren für ein Aminoalkyl(meth)acrylat, das die folgenden aufeinanderfolgenden Schritte umfasst:

1) Ansetzen einer Mischung M1, das Folgendes umfasst:

(i) von 50 bis 97,899 Gew.% zumindest eines $Akyl_1$(meth)acrylatesters;
(ii) von 0,1 bis 40 Gew.% zumindest eines Alkanolamins;
(iii) von 0,001 und 10 Gew.% zumindest eines organometallischen Katalysators;
(iv) von 2 bis 30 Gew.% zumindest einer Lösung;

bezogen auf das Gesamtgewicht der Mischung M1;
2) Ansetzen einer Mischung M2 durch:

(i) Erwärmen der Mischung M1 auf eine Temperatur T1 zwischen 40 °C und 120 °C und Abtrennen des $Alkyl_1$-Alkohols, der sich während der Reaktion bildet, durch azeotrope Destillation; die azeotrope Destillation wird zwischen 30 bis 720 Minuten bei T1 durchgeführt,
(ii) weitere kontinuierliche Zugabe während der azeotropen Destillation einer Menge des mindestens einen Alkanolamins zwischen 15 und 85 Gew.% bezogen auf die Gesamtmenge des in den Schritten 1) und 2) verwendeten Alkanolamins;

3) Gewinnen des Aminoalkyl(meth)acrylats durch Destillation der Mischung M2, wobei das Amin des Alkanolamins ein tertiäres Amin $-NR_1R_2$ ist, worin $R_1$ und $R_2$, die identisch oder unterschiedlich sein können, Alkylgruppen sind, die zwischen 1 und 8 Kohlenstoffatomen umfassen, und das $Alkyl_1$(meth)acrylatester linear ist und die $Alkyl_1$-Kette zwischen 1 und 10 Kohlenstoffen umfasst.

**2.** Batch-Syntheseverfahren nach Anspruch 1, worin das $Alkyl_1$(meth)acrylatester Methyl(meth)acrylat ist.

**3.** Batch-Syntheseverfahren nach einem der Ansprüche 1 bis 2, worin das Alkanolamin Dimethylaminoethanol ist.

**4.** Batch-Syntheseverfahren nach einem der Ansprüche 1 bis 3, worin der Katalysator die Form einer Suspension S1 einnimmt, in welcher 70 bis 95 Gew.% des Katalysators gelöst sind und 5 bis 30 Gew.% des Katalysators die Form von Teilchen einnehmen, die in der Suspension S1suspendiert sind, bezogen auf die Gesamtmenge des Katalysators in der Suspension S1.

**5.** Batch-Syntheseverfahren nach einem der Ansprüche 1 bis 4, worin die Menge an Alkanolamin, das in Schritt 2)(ii) zugegeben wird, zwischen 20 und 80 Gew.%, bezogen auf das Gesamtgewicht des in den Schritten 1) und 2) verwendeten Alkanolamins, umfasst.

**6.** Batch-Syntheseverfahren nach einem der Ansprüche 1 bis 5, worin in Schritt 2)(ii), das Alkanolamin kontinuierlich zugegeben wird, während der $Alkyl_1$-Alkohol verdampft, und die Zugabe zwischen 30 und 720 Minuten dauert.

**7.** Batch-Syntheseverfahren nach einem der Ansprüche 1 bis 6, worin eine zusätzliche Menge an Katalysator kontinuierlich in Schritt 2)(ii) zugegeben wird.

**8.** Batch-Syntheseverfahren nach Anspruch 7, worin der Katalysator kontinuierlich in einer Menge zwischen 1 und 80 Gew.% bezogen auf das Gesamtgewicht des in den Schritten 1) und 2) verwendeten Katalysators zugegeben wird.

**9.** Batch-Syntheseverfahren nach einem der Ansprüche 1 bis 8, worin Schritt 2)(ii) initiiert wird, wenn die Mischung M1 ihren Siedepunkt erreicht.

**10.** Batch-Syntheseverfahren nach einem der Ansprüche 1 bis 9, worin Schritt 2)(ii) initiiert wird, wenn der $Alkyl_1$-Alkohol zu verdampfen beginnt.

**11.** Batch-Syntheseverfahren nach einem der Ansprüche 1 bis 10, worin die Schritte 1 bis 3 wiederholt werden.

**12.** Batch-Syntheseverfahren nach einem der Ansprüche 1 bis 10, worin die Schritte 1 bis 3 wiederholt werden und worin das Batch-Syntheseverfahren mit der Rückgewinnung des Katalysators verbunden ist.

**13.** Batch-Syntheseverfahren nach einem der Ansprüche 1 bis 12, worin zumindest eines aus dem $Alkyl_1$(meth)acrylatester, dem Alkanolamin und dem Lösungsmittel aus einem Ausgangsmaterial stammt, das recyceltes Material oder Materialien biologischen Ursprungs umfasst.

**Revendications**

1. Procédé de synthèse par lots d'un (méth)acrylate d'aminoalkyle comprenant les étapes successives suivantes :

    1) Préparation d'un mélange M1 comprenant :

    (i) de 50 à 97,899 % en poids d'au moins un ester de (méth)acrylate d'alkyle$_1$ ;
    (ii) de 0,1 à 40 % en poids d'au moins une alcanolamine ;
    (iii) de 0,001 à 10 % en poids d'au moins un catalyseur organométallique ;
    (iv) de 2 à 30 % en poids d'au moins un solvant ;

    sur la base du poids total du mélange M1 ;
    2) préparer un mélange M2 par :

    (i) chauffage du mélange M1 à une température T1 comprise entre 40°C et 120°C et élimination de l'alcool alkyle$_1$ qui se forme au cours de la réaction par distillation azéotropique ; la distillation azéotropique est effectuée pendant 30 à 720 min à T1,
    (ii) en outre, addition en continu, pendant la distillation azéotropique, d'une quantité de la au moins une alcanolamine comprise entre 15 et 85 % en poids sur la base de la quantité totale d'alcanolamine utilisée dans les étapes 1) et 2) ;

    3) récupération du (méth)acrylate d'aminoalkyle par distillation du mélange M2, l'amine de l'alcanolamine est une amine tertiaire - $NR_1R_2$ dans laquelle $R_1$ et $R_2$, identiques ou différents, sont des groupes alkyle comprenant entre 1 et 8 atomes de carbone et l'ester de (méth)acrylate d'alkyle$_1$ est linéaire et la chaîne d'alkyle$_1$ comprend entre 1 et 10 atomes de carbone.

2. Procédé de synthèse par lots selon la revendication 1, dans lequel l'ester de (méth)acrylate d'alkyle, est le (méth) acrylate de méthyle.

3. Procédé de synthèse par lots selon l'une quelconque des revendications 1 à 2, dans lequel l'alcanolamine est le diméthylaminoéthanol.

4. Procédé de synthèse par lots selon l'une quelconque des revendications 1 à 3, dans lequel le catalyseur est sous la forme d'une suspension S1 dans laquelle 70 % à 95 % en poids du catalyseur sont solubilisés, et 5 % à 30 % en poids du catalyseur se présentent sous la forme de particules mises en suspension dans la suspension S1, sur la base de la quantité totale de catalyseur dans la suspension S1.

5. Procédé de synthèse par lots selon l'une quelconque des revendications 1 à 4, dans lequel la quantité d'alcanolamine ajoutée dans l'étape 2)(ii) est comprise entre 20 et 80 % en poids sur la base du poids total d'alcanolamine utilisé dans les étapes 1) et 2).

6. Procédé de synthèse par lots selon l'une quelconque des revendications 1 à 5, dans lequel dans l'étape 2)(ii), l'alcanolamine est ajoutée en continu pendant que l'alcool d'alkyle$_1$ s'évapore, et l'addition dure entre 30 et 720 min.

7. Procédé de synthèse par lots selon l'une quelconque des revendications 1 à 6, dans lequel une quantité supplémentaire de catalyseur est ajoutée en continu à l'étape 2)(ii).

8. Procédé de synthèse par lots selon la revendication 7, dans lequel le catalyseur est ajouté en continu en une quantité comprise entre 1 et 80 % en poids sur la base du poids total du catalyseur utilisé dans les étapes 1) et 2).

9. Procédé de synthèse par lots selon l'une quelconque des revendications 1 à 8, dans lequel l'étape 2)(ii) est initiée lorsque le mélange M1 atteint son point d'ébullition.

10. Procédé de synthèse par lots selon l'une quelconque des revendications 1 à 9, dans lequel l'étape 2)(ii) est initiée lorsque l'alcool d'alkyle, commence à s'évaporer.

11. Procédé de synthèse par lots selon l'une quelconque des revendications 1 à 10, dans lequel les étapes 1 à 3 sont répétées.

**12.** Procédé de synthèse par lots selon l'une quelconque des revendications 1 à 10, dans lequel les étapes 1 à 3 sont répétées et dans lequel le procédé de synthèse par lots implique un recyclage du catalyseur.

**13.** Procédé de synthèse par lots selon l'une quelconque des revendications 1 à 12, dans lequel au moins un parmi l'ester de $_1$ (méth)acrylate d'alkyle, l'alcanolamine et le solvant provient d'une matière première comprenant un matériau recyclé ou des matériaux d'origine biologique.

**EP 4 680 594 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019196048 A
- US 4059617 A
- WO 2013045786 A
- WO 2016124837 A
- CN 117000313